# EUROPEAN PATENT APPLICATION

(11) **EP 1 216 709 A1**
(43) Date of publication of application: **26.06.2002**
(21) Application number: 00128252.4
(22) Date of filing: 21.12.2000
(51) Int. Cl.: A61K 38/48, A61P 7/04

(54) **Use of activated coagulation factor VII for treating thrombolytic therapy-induced major bleedings**

(71) Applicant: Boehringer Ingelheim Pharma KG, 55216 Ingelheim am Rhein (DE)
(72) Inventor: Skamira, Cord, 88444 Ummendorf (DE); Stassen, Jean Marie, 88422 Bad Buchau (DE); Heusel, Gerhard, 88444 Ummendorf (DE); Wienen, Wolfgang, 88400 Biberach (DE)
(74) Representative: Barz, Peter, Dr.

(57) **Abstract**

Major bleedings induced by thrombolytic/fibrinolytic therapy, including intracranial haemorrhages, are treated by administering to a subject suffering from such bleedings an effective amount of activated coagulation factor VII (VIIa) or of a functional derivative thereof.

## Description

### Background of the Invention

The haemostatic system, including blood platelets, blood coagulation and fibrinolysis, plays a crucial role in maintaining blood flow and limiting blood loss upon vascular injury. Upon vascular injury, blood platelets adhere to the vessel wall, aggregate and form a plug which is consolidated by a fibrin network formed upon activation of the coagulation via activation of factor VII, factor X and prothrombin. After cessation of the blood loss, wound healing is initiated, followed by dissolution of the blood clot by the fibrinolytic system via tissue plasminogen activator (t-PA)-induced plasmin generation.

During pathological situations, as in cardiovascular diseases, this system can escape the normal physiological regulation and result in a complete occlusion of the blood vessel as is observed after the rupture of an atherosclerotic plaque. To treat this thrombotic obstruction and to restore blood flow, thrombolytic therapy is widely used (Ref.1).

Thrombolytic therapy comprises a combination treatment with an antiplatelet agent (e. g. acetylsalicylic acid), an anticoagulant agent (e.g. heparin) and a fibrinolytic agent (e.g. a tissue plasminogen activator, streptokinase, staphylokinase, urokinase or a derivative thereof). The combination of these agents, although therapeutically very effective, also involves the significant risk of inducing bleeding complications including haemorrhagic stroke (Refs.2,3). Severe bleeding occurs in a significant number of patients subjected to thrombolytic therapy. According to the most recent large clinical trial in thrombolytic therapy, i.e. the Assent-2 trial involving 16949 patients, the rates of intracranial haemorrhage are 0.93% for tenecteplase (a tissue plasminogen activator (t-PA) derivative) and 0.94% for alteplase (also a t-PA derivative), and non-cerebral bleeding complications are 26.43 and 28.95% for tenecteplase and for alteplase, respectively. The need for blood transfusion was 4.25 and 5.49%. The rate of death or non-fatal stroke at 30 days was 7.11% with tenecteplase and 7.04% with alteplase (Ref.3), respectively.

Thus, there is a need for a medicament which provides the physician with an antidote to reverse such thrombolytic therapy-induced major bleedings independent of the thrombolytic drug used.

### Summary of the Invention

It is therefore an object of the present invention to provide a composition and a method for treating thrombolytic/fibrinolytic therapy-induced major bleedings in a subject suffering from such bleedings, for instance, in humans or animals.

The present invention is based on the discovery that this object can be solved by using activated coagulation factor VII (VIIa) for this purpose.

Factor VII is a vitamin K dependent glycoprotein which is physiologically synthesized by liver cells and secreted into the blood as a single-chain molecule consisting of 406 amino acid residues. The activation of factor VII to factor VIIa involves the hydrolysis of a single peptide bond between Arg-152 and IIe-153, resulting in a two-chain molecule consisting of a light chain of 152 amino acid residues and a heavy chain of 254 amino acid residues held together by a single disulfide bond. In its activated form, the protein acts as a serine protease that participates in the extrinsic pathway of the blood coagulation cascade. Upon exposure of tissue factor (TF) at the damaged vascular wall, a complex with factor VII is formed resulting in activated factor VII (factor VIIa), the complex of TF and factor VIIa activates factor X to factor Xa and in turn converts prothombin into thrombin. Thrombin plays a central role in the blood coagulation and the wound healing. In the initial phase of vascular injury it induces platelet aggregation and fibrin formation followed by the stimulation of cell growth to enhance the repair of the damaged blood vessel (Ref.1).

Recombinant factor VII (rFVII) is expressed in baby hamster kidney cells after cellular transfection with the human DNA encoding for factor VII and converted into activated factor VII (rFVIIa) during purification (Ref.4). Recombinant factor VIIa in its marketed form, NovoSeven® (Novo Nordisk, Bagsvaerd, Denmark), has been increasingly used in the treatment of bleeding episodes in a wide range of bleeding disorders, predominantly haemophilia patients with inhibitors against factor VII or IX, where other therapies were ineffective (Refs.5-10).

However, in spite of some 13 years experience with this drug, the use of factor VIIa to reverse thrombolytic therapy-induced major bleeding events has never been suggested or investigated.

### Detailled Description of the Invention

In a first aspect, the present invention relates to the use of activated coagulation factor VII (VIIa) or a functional derivative thereof in the manufacture of a medicament for the treatment of thrombolytic/fibrinolytic therapy-induced major bleedings, including intracranial haemorrhages.

In a second aspect, the present invention relates to a method for treating thrombolytic/fibrinolytic therapy-induced major bleedings, including intracranial haemorrhages, which method comprises administering to a subject suffering from such bleedings an effective amount of activated coagulation factor VII (VIIa) or of a functional derivative thereof. The method is particularly useful in the treatment of mammalians, including humans.

Human purified factor VIIa suitable for use in the present invention may be isolated from natural sources or, preferably, made by recombinant DNA techniques, e.g. as described in Ref.20. Factor VIIa produced by recombinant techniques may be essentially identical to the native factor VIIa, such as the product NovoSeven® from Novo Nordisk. The term "functional derivative" refers to a modified derivative of factor VIIa having substantially the same biological activity of interest. Such functional factor VIIa derivatives may be produced, for instance, by site-specific mutagenesis of the nucleic acid sequence encoding factor VII resulting in recombinant proteins wherein one ore more amino acid residues of the naturally occurring amino acid sequence have been substituted, inserted or deleted. Also, useful post-translational modifications may be effected, for instance, changes in the glycosylation pattern.

The thrombolytic/fibrinolytic drug used in thrombolytic/fibrinolytic therapy may comprise any form of a native or recombinant tissue plasminogen activator such as alteplase or reteplase, duteplase, saruplase, recombinant DSPA alpha 1 (BAT PA), streptokinase, anistreplase, staphylokinase, including pegilated staphylokinase and mutants of staphylokinase having no or reduced immunogenicity, urokinase, single-chain urokinase, any of the third generation thrombolytic agents known in the art, e.g. amediplase, tenecteplase, monteplase, lanoteplase, pamiteplase (Refs. 2,3,11-15,19), or any therapeutically acceptable derivative thereof.

Preferably, the medicament or pharmaceutical composition comprising factor VIIa will be administered via intravenous bolus injection or via intermittent or continuous intravenous infusion. Also, a combination of a single intravenous bolus injection followed by intravenous infusion of factor VIIa may be useful.

Suitable pharmaceutical preparations for injection or infusion purposes include sterile aqueous solutions and sterile powders for the extemporaneous preparation of sterile injectable or infusable solutions. Generally, the final solutions will also contain suitable salts and other auxiliary agents as known in the art. For example, a reconstituted aqueous solution of NovoSeven® as produced and sold by Novo Nordisk comprises 3 mg/ml sodium chloride, 1,5 mg/ml calcium chloride dihydrate, 1,3 mg/ml N-glycylglycine, 0,1 mg/ml polysorbate 80 and 30 mg/ml mannitol.

The dosage of factor VIIa to be administered will vary, depending on, e.g., age and physical condition of the particular subject, the severity of the bleeding complications to be treated, and the selected route of administration. The appropriate dosage can be readily determined by a person skilled in the art.

Typically, a suitable dosage range of factor VIIa for intravenous bolus injection will be from about 3000-6000 IU (International Units; in accordance with the first international standard relating to factor VIIa 89/688), corresponding to about 60-120 µg recombinant factor VIIa, per kg body weight. Preferably, the dosage for intravenous bolus injection will range from about 4500-6000 IU per kg body weight. Since usually the systemic half-life of recombinant factor VIIa is only about 2-3 h, repeated intravenous bolus injections in relatively short time intervals, preferably in intervals of 2-3 h, more preferably 2 h, may be necessary. Where appropriate, initial time intervals may be extended up to e.g. 4, 6, 8, 12 h in the course of the treatment.

Where therapeutically appropriate, the intravenous bolus injection should be administered within about 2-5 minutes.

A suitable dosage regimen for intermittent infusion of factor VIIa may be about 4500-6000 IU per kg body weight every 2-6 hours.

A suitable dosage of factor VIIa for continuous infusion may be about 500 - 1500 IU/kg/h, corresponding to about 10-30 µg rFVIIa/kg/h. Where appropriate, a single bolus injection dosage of about 4500-6000 IU per kg body weight may precede the continuous infusion of factor VIIa. Results from previous studies on continuous infusion of recombinant factor VIIa (Refs.17,18) indicate that treatment by continuous infusion of factor VIIa will be effective at lower total dosages of the drug, compared with bolus injections.

Since up to date the frequency of side effects in known applications of factor VIIa is low and biochemical signs indicating thrombogenicity or consumption coagulopathy are absent, it it is expected that major bleedings related to thrombolytic therapy are effectively reversed by activated factor VIIa without apparent harm to the recipient.

### References

1. Stassen JM, Nyström Å. A historical review on thrombosis and hemostasis. Ann Plast Surgery 1997; 39: 317-329.
2. The GUSTO investigators. An international randomized trial comparing four thrombolytic strategies for acute myocardial infarction. N Engl J Med. 1993 Sep 2;329(10):673-82.
3. Assent-2. Single-bolus tenecteplase compared with front-loaded alteplase in acute myocardial infarction: the ASSENT-2 double-blind randomised trial. Assessment of the Safety and Efficacy of a New Thrombolytic Investigators. Lancet 1999 Aug 28;354(9180):716-22
4. Thim L, Bjoern S, Christensen M, Nicolaisen EM, Lund-Hansen T, Pedersen AH, Hedner U. Amino acid sequence and posttranslational modifications of human factor VIIa from plasma and transfected baby hamster kidney cells. Biochemistry. 1988;27:7785-93.
5. Macik BG, Hohneker J, Roberts HR, Griffin AM. Use of recombinant activated factor VII for treatment of a retropharyngeal hemorrhage in a hemophilic patient with a high titer inhibitor. Am J Hematol. 1989;32:232-4.
6. Kenet G, Walden R, Eldad A, Martinowitz U. Treatment of traumatic bleeding with recombinant factor VIIa. Lancet 1999;354:1879.
7. White B, McHale J, Ravi N, Reynolds J, Stephens R, Moriarty J, Smith OP. Successful use of recombinant FVIIa (NovoSeven®) in the management of intractable post-surgical intra-abdominal haemorrhage. Br J Haematol 1999;107:677-8.
8. Al Douri M, Shafi T, Al Khudairi D, Al Bokhari E, Black L, Akinwale N, Osman Musa M, Al Homaidhi A, Al Fagih M, Borum Andreasen R. Effect of the administration of recombinant activated factor VII (rFVIIa; NovoSeven®) in the management of severe uncontrolled bleeding in patients undergoing heart valve replacement surgery. Blood Coagul Fibrinolysis. 2000 Suppl 1:S121-7.
9. Negrier C, Lienhart A, Overall experience with NovoSeven®. Blood Coagul Fibrinolysis 2000 Suppl 1:S19-24.
10. Wong WY, Huang WC, Miller R, McGinty K, Whisnant JK Clinical efficacy and recovery levels of recombinant FVIIa (NovoSeven®) in the treatment of intracranial haemorrhage in severe neonatal FVII deficiency. Haemophilia 2000;6:50-4.
11. Gissi International Study Group. In-hospital mortality and clinical course of 20,891 patients with suspected acute myocardial infarction randomised between alteplase and streptokinase with or without heparin. The Lancet. 1990;336:71-5.
12. The Global Use of Strategies to Open Occluded Coronary Arteries (GUSTO III) Investigators. A comparison of reteplase with alteplase for acute myocardial infarction. N Engl J Med. 1997;337:1118-2.
13. Van de Werf F, Cannon CP, Luyten A, Houbracken K, McCabe CH, Berioli S, Bluhmki E, Sarelin H, Wang-Clow F, Fox NL, Braunwald E. Safety assessment of single-bolus administration of TNK tissue-plasminogen activator in acute myocardial infarction: the ASSENT-1 trial. The ASSENT-1 Investigators. Am Heart J. 1999;137:786-91.
14.Collen D, Sinnaeve P, Demarsin E, Moreau H, De Maeyer M, Jespers L, Laroche Y, Van De Werf F. polyethylene glycol-derivatized cystein-substitution variants of recombinant staphylokinase for single-bolus treatment of acute myocardial infarction. Circulation. 2000;102:1766-72.
15.Laroche Y, Heymans S, Capaert S, De Cock F, Demarsin E, Collen D. Recombinant staphylokinase variants with reduced antigenicity due to elimination of B-lymphocyte epitopes. Blood. 2000;96:1425-32.
16.Hedner U. Dosing and Monitoring NovoSeven® treatment, Haemostasis 1996; 26 (suppl 1):102-108.
17.Schulman S, Bech Jensen M, Varon D, Keller N, Gitel S, Horoszowski H, Heim M, Martinowitz U. Feasibility of using recombinant factor VIIa in continuous infusion. Thromb Haemost. 1996 Mar;75(3):432-6.
18. Baudo F, Redaelli R, Caimi TM, Mostarda G, Somaini G, de Cataldo F. The continuous infusion of recombinant activated factor VIIa (rFVIIa) in patients with factor VIII inhibitors activates the coagulation and fibrinolytic systems without clinical complications. Thromb Res. 2000;99:21-4.
19. Verstraete M. Third-generation thrombolytic drugs. Am J Med. 2000;109:52-8.
20. Hagen et al., Proc. Natl. Acad.Sci. USA 1986;83: 2412-2416.

## Claims

1. The use of activated coagulation factor VII (VIIa) or of a functional derivative thereof in the manufacture of a medicament for the treatment of thrombolytic/fibrinolytic therapy-induced major bleedings.

2. The use of claim 1 wherein the bleedings are induced by thrombolytic/fibrinolytic therapy using any form of a native or recombinant tissue plasminogen activator such as alteplase or reteplase, duteplase, saruplase, recombinant DSPA alpha 1 (BAT PA), streptokinase, staphylokinase, including pegilated staphylokinase and mutants of staphylokinase having no or reduced immunogenicity, urokinase, single-chain urokinase, third generation thrombolytic agents, or a therapeutically acceptable derivative thereof.

3. The use of claim 1 or 2 wherein the bleedings are intracranial haemorrhages.

4. The use of any one of claims 1-3 wherein the medicament is suitable for administration of an effective amount of factor VIIa or a functional derivative thereof via intravenous bolus injection or intravenous infusion.

5. The use of claim 4 wherein the effective amount of factor VIIa or a functional derivative thereof comprises one or more single intravenous bolus injection dosages of about 3000-6000 IU, corresponding to about 60-120 µg recombinant factor VIIa, per kg body weight and/or an intravenous infusion dosage of about 500-1500 IU, corresponding to about 10-30 µg recombinant factor VIIa, per kg body weight per hour.

6. The use of claim 4 or 5 wherein the effective amount of factor VIIa or a functional derivative thereof is administered in the form of repeated intravenous bolus injections in time intervals of about 2 hours.

7. A method for treating thrombolytic/fibrinolytic therapy-induced major bleedings, including intracranial haemorrhages, which method comprises administering to a subject suffering from such bleedings an effective amount of activated coagulation factor VII (VIIa) or of a functional derivative thereof.

8. The method of claim 7 wherein the bleedings are induced by thrombolytic/fibrinolytic therapy using any form of a native or recombinant tissue plasminogen activator such as alteplase or reteplase, duteplase, saruplase, recombinant DSPA alpha 1 (BAT PA), streptokinase, staphylokinase, including pegilated staphylokinase and mutants of staphylokinase having no or reduced immunogenicity, urokinase, single-chain urokinase, third generation thrombolytic agents, or a therapeutically acceptable derivative thereof.

9. The method of claim 7 or 8 wherein an effective amount of factor VIIa or a functional derivative thereof is administered via intravenous bolus injection or intravenous infusion, preferably via repeated intravenous bolus injections in time intervals of about 2 hours.

10. The method of claim 9 wherein the effective amount of factor VIIa or a functional derivative thereof comprises one or more single intravenous bolus injection dosages of about 3000-6000 IU, corresponding to about 60-120 µg recombinant factor VIIa, per kg body weight and/or an intravenous infusion dosage of about 500-1500 IU, corresponding to about 10-30 µg recombinant factor VIIa, per kg body weight per hour.
